(19) Europäisches Patentamt
European Patent Office
Office européen des brevets

(11) Publication number: **0 163 367 B1**

(12) **EUROPEAN PATENT SPECIFICATION**

(45) Date of publication of patent specification: **19.08.92**

(51) Int. Cl.[5]: **G01N 33/62**

(21) Application number: **85301531.1**

(22) Date of filing: **06.03.85**

The file contains technical information submitted after the application was filed and not included in this specification

(54) **Diagnostic reagent for assay of urea nitrogen.**

(30) Priority: **30.03.84 US 595145**

(43) Date of publication of application:
**04.12.85 Bulletin 85/49**

(45) Publication of the grant of the patent:
**19.08.92 Bulletin 92/34**

(84) Designated Contracting States:
**BE CH DE FR GB IT LI NL SE**

(56) References cited:
**US-A- 4 040 787**
**US-A- 4 357 144**

(73) Proprietor: **MILES INC.(an Indiana corp.)**
**511 Benedict Avenue**
**Tarrytown, New York 10591-5097(US)**

(72) Inventor: **Klotzsh, Sigrid G.**
**10 Cayuga Lane**
**Irvington, NY 10533(US)**
Inventor: **Vadaszy, Richard**
**99 Lake Road**
**Valley Cottage, NY 10989(US)**

(74) Representative: **Wain, Christopher Paul et al**
**A.A. THORNTON & CO. Northumberland House 303-306 High Holborn**
**London WC1V 7LE(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 163 367 B1

## Description

This invention relates generally to urea assay methodology and, more particularly, to a colorimetric urea nitrogen reagent and its use.

Essentially three groups of methods have been used for the determination of urea nitrogen (UN). They can be classified as miscellaneous methods, methods based on the reaction with diacetyl-monoxime or similar compounds, and methods employing urease. Methods employing diacetyl-monoxime and urease have become the most popular.

In 1939, Fearon (Biochem. J. 33:902-907 (1930)) found that the reaction of diacetyl monoxime (DAM) followed by oxidation produced colors with compounds of the formula $R_1$-NH-CO-NH$R_2$ when $R_1$ is either H or a single aliphatic radical. $R_2$ is not an acyl radical and is usually hydrogen. Colors were produced with urea, creatinine, methylurea (and urea derivatives), allantoin, and proteins. Many urea substitutes yielded a red pigment, but only urea produced a yellow color. (Koritz et al, J. Bio. Chem. 209:145-150 (1954)).

In 1942, Ormbsy (J. Biol. Chem. 146:595-604 (1942)) applied the diacetyl-monoxime (DAM) reaction in a strongly acidic medium to the determination of urea. The resultant color was intensified by oxidation with potassium persulfate. Other oxidants have been used and are also described in the literature.

Natelson, Scott and Beffa (Am. J: Clin. Pathol. 21:275-281 (1951)) obviated the need for oxidizing agents by use of free diacetyl rather than the derivative.

In 1963, Coulombe and Favreau (Clin. Chem. 9:102-108 (1963)) demonstrated the effect of thiosemicarbazide upon the direct reaction between diacetyl-monoxime and urea. This compound further intensified the color and inhibited photo-sensitivity. Subsequently, the semicarbazide reagent was incorporated in an automated procedure described by Pellerin (Clin. Chem. 10:374-375 (1964)).

In 1965, Marsh, Fingerhut, and Miller (Clin. Chem. 11:624-627 (1965)) presented both an automated and a manual diacetyl-monoxime method for direct determination of urea. Both methods proved highly sensitive due to the combined use of thiosemicarbazide and ferric ions.

In spite of the above advances, however, such urea determinations have been plagued by instability of working solutions resulting in limited shelf life and potentially erroneous results.

We have now devised a diagnostic reagent for this colorimetric determination, which reagent has good storage stability, and which provides enhanced sensitivity and linearity when employed in urea nitrogen assays to give rapid and precise results.

According to the present invention, there is provided a storage stable diagnostic reagent solution useful for the colorimetric determination of urea nitrogen which solution comprises:

(a) from 0.02% to 5.0% w/v of diacetyl monoxime or a derivative thereof,

(b) from 0.001% to 0.2% w/v of a color enhancer; and

(c) up to 0.5% w/v of a compound of the formula:

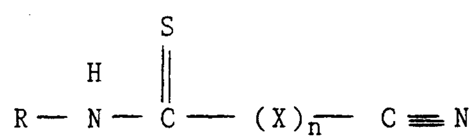

wherein R is an alkyl or amino, mono- or dialkylamino group, said alkyl(s) having from 1 to 4 carbons; X is imino; n is 0, 1 or 2; or a thio salt thereof.

A preferred color enhancer is thiosemicarbarzide. Preferred component (c) compounds are those of the formulae

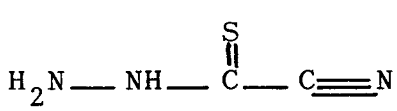

and

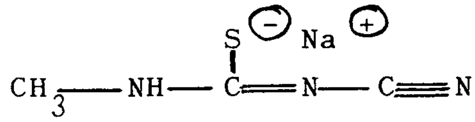

The reagents of the invention (especially the preferred reagents) may be used in colorimetric determinations of urea nitrogen in a urea-containing sample whereby the urea concentration of the sample is linearly related to a colored reaction product colorimetrically determinable at 520 nm.

As described earlier, Coulombe and Favreau demonstrated the effect of thiosemicarbazide upon the direct reaction between diacetyl monoxime and urea, namely intensified color. However, urea nitrogen color reagents comprising diacetyl monoxime and thiosemicarbazide are unstable. It is the need to provide a reagent which is storage stable, i.e. stable for at least 6 months at room temperature, and which still has full sensitivity and linearity, which the present invention is intended to meet. As used herein, "sensitivity" is defined as about 0.0022 absorbance units per mg. urea nitrogen/dl and "linearity" is defined as linear response to test samples in the range of 0-150 mg. urea nitrogen/dl.

The reagents of the present invention, may be used for colorimetrically assaying the urea content of fluids such as blood serum, plasma, urine and spinal fluid.

The chromogenic agent is preferably diacetyl, although any derivative thereof, e.g. diacetyl monoxime, may be used so long as the required ureide-group reaction takes place to provide a measurable yellow color. It is preferred to have from 0.02% to 5.0% w/v of said chromogenic agent in the reagent with from 0.1% to 0.3% w/v most preferred.

Whilst, as previously stated, the color enhancer is preferably thiosemicarbazide, any other material can be used which intensifies the aforedescribed color and change of hue from yellow to red. It is preferred to have from 0.001% w/v to 0.2% w/v of said color enhancer, and most preferred is a range from 0.002% to 0.5% w/v.

It is preferred to have up to 0.5% w/v of the component (c) compound in the reagent composition, the most preferred amount being in the range of from 0.02% to 0.1% w/v.

The invention also includes a colorimetric assay for determining the urea content of fluids, in which assay there is employed a diagnostic reagent of the invention. Urea is the chief end product of protein metabolism in the blood, and its concentration in the blood provides an indicator of kidney function. The quantitative testing for urea is therefore an invaluable tool in the analysis of kidney malfunction.

Although the most preferred application of the method of this invention is to blood serum, it is to be understood that the method is applicable to other body fluids as well, such as blood plasma, urine, spinal fluid, or indeed to any aqueous solution containing urea.

In order that the invention may be more fully understood, the following Examples are given by way of illustration only.

## EXAMPLE 1

This Example describes the use of a composition of the invention in determining blood urea nitrogen on a SMAC analytical system. (SMAC is a registered trademark.) The SMAC system was used in this Example in accordance with the written instructions available from the manufacturer, TECHNICON INSTRUMENTS CORPORATION, Tarrytown, New York 10591.

Composition of Reagents:

| A. BUN Sample Diluent | |
|---|---|
| deionized water<br>surfactant (a suitable surfactant is a 30% aq. solution of Brij. -35, a trademark of ICI Americas, Inc., Wilmington, Delaware) | 1.0 1<br>0.001 1 |

| B. BUN Color Reagent | |
|---|---|
| deionized water | 1.0 1 |
| diacetyl monoxime | 2.0 g |
| thiosemicarbazide | 0.2 g |
| 1-cyano-3-methylisothiourea, sodium salt surfactant and other non-active ingredients | 0.6 g |

| C. BUN Acid Reagent | |
|---|---|
| sulfuric acid, conc. | 0.25 1 |
| phosphoric acid, conc. | 0.0012 1 |
| ferric chloride, hexahydrate | 0.06 g |
| deionized water | qs to 1 1 |

A serum sample is introduced into the system and diluted in an air-segmented stream of BUN sample diluent to a ratio of 74 µl sample/287 µl diluent. After mixing, the diluted sample is exposed to a dialysis membrane. The urea dialyzes across the membrane into 385 µl of a steady stream of BUN color reagent. The analyte-containing BUN color reagent stream is then acidified with 226 µl BUN acid reagent. After acidifying by addition of BUN acid reagent, the stream of reaction mixture is heated to 90 ° C at which point, the thiosemicarbazide intensifies the color. The absorbance of the reaction mixture stream is measured at 520 nm in a flowcell. The observed absorbance is proportional to the urea nitrogen in the sample and the urea nitrogen concentration is calculated by comparison with an appropriate standard.

## Claims

**1.** A storage-stable diagnostic reagent solution useful for the colorimetric determination of urea nitrogen in a urea-containing sample, which solution comprises:
(a) from 0.02% to 5.0% w/v of diacetyl monoxime or a derivative thereof,
(b) from 0.001% to 0.2% w/v of a color enhancer; and
(c) up to 0.5% w/v of a compound of the formula:

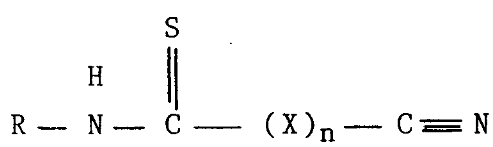

wherein R is an alkyl or amino, mono- or dialkylamino group, said alkyl(s) having from 1 to 4 carbons; X is imino; n is 0, 1 or 2; or a thio salt thereof.

**2.** A reagent according to claim 1, wherein said color enhancer is thiosemicarbazide.

**3.** A reagent according to claim 1 or 2, wherein component (c) is a compound of the formula:

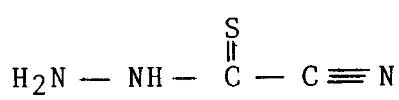

**4.** A reagent according to claim 1 or 2, wherein component (c) is a compound of the formula:

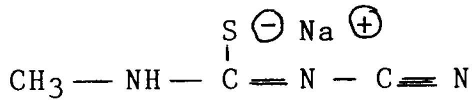

5. A colorimetric method for the determination of urea nitrogen in a urea-containing sample which employs the diagnostic reagent of any of claims 1 to 4.

## Patentansprüche

1. Haltbare diagnostische Reagenzlösung, verwendbar für die kolorimetrische Bestimmung von Harnstoff-Stickstoff in einer Harnstoff enthaltenden Probe, wobei die Lösung enthält:
   (a) 0,02 bis 5,0% (Gewicht/Volumen) von Diacetyl-monoxim oder einem Derivat davon,
   (b) 0,001 bis 0,2% (Gewicht/Volumen) eines Farbverstärkers und
   (c) bis 0,5% Gewicht/Volumen) einer Verbindung der Formel

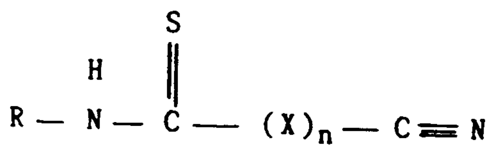

   worin R eine Alkyl-, Amino- oder Mono- oder Dialkylaminogruppe bedeutet, wobei die Alkylgruppe(n) 1 bis 4 Kohlenstoffatome aufweist bzw. aufweisen, X eine Iminogruppe bedeutet und n 0,1 oder 2 ist, oder eines Thiosalzes davon.

2. Reagenz gemäß Anspruch 1, worin der Farbverstärker Thiosemicarbazid ist.

3. Reagenz gemäß Anspruch 1 oder 2, worin die Komponente (c) eine Verbindung der Formel

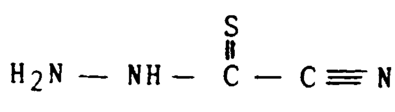

   ist.

4. Reagenz gemäß Anspruch 1 oder 2, worin die Komponente (c) eine Verbindung der Formel

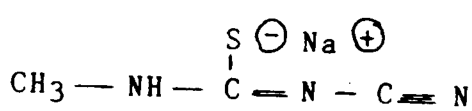

   ist.

5. Kolorimetrisches Verfahren zur Bestimmung von Harnstoff-Stickstoff in einer Harnstoff enthaltenden Probe, wobei das diagnostische Reagenz gemäß einem der Ansprüche 1 bis 4 verwendet wird.

## Revendications

1. Solution de réactif de diagnostic stable au stockage, pouvant être utilisée pour la détermination colorimétrique de l'azote uréique dans un échantillon contenant de l'urée, laquelle solution contient :
   (a) de 0,02 % à 5,0 % (p/v) de diacétyl-monoxime ou d'un dérivé de celle-ci,
   (b) de 0,001 % à 0,2 % (p/v) d'un renforçateur de couleur ; et
   (c) jusqu'à 0,5 % (p/v) d'un composé répondant à la formule :

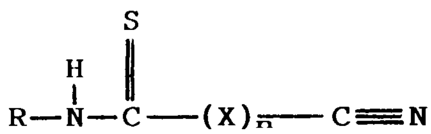

où R représente un groupe alcoyle ou amino, mono- ou dialcoylamino, le ou les groupe(s) alcoyle comportant 1 à 4 atome(s) de carbone ; X représente un groupe imino ; n vaut 0, 1 ou 2 ; ou un sel thio de celui-ci.

**2.** Réactif selon la revendication 1, où ledit renforçateur de couleur est le thiosemicarbazide.

**3.** Réactif selon la revendication 1 ou 2, où le composant (c) est un composé répondant à la formule :

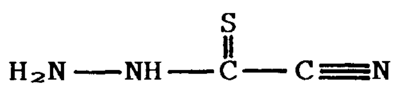

**4.** Réactif selon la revendication 1 ou 2, où le composant (c) est un composé répondant à la formule :

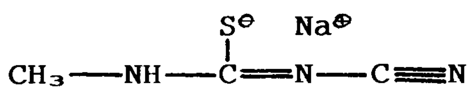

**5.** Procédé colorimétrique pour la détermination de l'azote uréique dans un échantillon contenant de l'urée, dans lequel on utilise le réactif de diagnostic selon une quelconque des revendications 1 à 4.